# EUROPÄISCHE PATENTSCHRIFT

(11) **EP 1 592 473 B1**
(45) Veröffentlichungstag und Bekanntmachung des Hinweises auf die Patenterteilung: **02.01.2008**
(21) Anmeldenummer: 04703390.7
(22) Anmeldetag: 20.01.2004
(51) Int. Cl.: A61M 25/00

(54) **AORTENKANÜLE**
AORTIC CANNULA
CANULE AORTIQUE

(30) Priorität: 30.01.2003 DE 10303744
(43) Veröffentlichungstag der Anmeldung: 09.11.2005
(73) Patentinhaber: Sorin Group Deutschland GmbH, 80939 München (DE)
(72) Erfinder: SIEVERS, Hans-Hinrich, 24119 Kronshagen (DE); GÖLLNER, Marcus, 80797 München (DE); WETZIG, Michael, 80339 München (DE)
(74) Vertreter: HOFFMANN EITLE
(86) Internationale Anmeldenummer: PCT/EP2004/000425
(87) Internationale Veröffentlichungsnummer: WO 2004/067073

(56) Entgegenhaltungen:
- EP-A- 0 705 617
- US-B1- 6 387 087

## Beschreibung

Die Erfindung betrifft eine Aortenkanüle, insbesondere zur Verwendung während einer Herzoperation, zum Zuführen von Fluid in die Aorta.

Im Stand der Technik sind die Probleme, die bei der Zuführung von Fluid, insbesondere Blut, in den Aortenbogen oder ein anderes Gefäß des menschlichen Körpers auftreten können, vielfach beschrieben worden. An dieser Stelle wird dazu verwiesen auf DE 196 05 864 A1, WO 99/56808 A1, DE 199 33 171 A1, EP 0 612 536 A1 und EP 0 705 617 A1. Hervorgerufen werden diese Probleme primär durch das aus der Kanüle austretende Fluid, wenn das Fluid aus einer schlauchartigen Kanüle am distalen Ende mit einer sehr hohen Geschwindigkeit gerichtet ausströmt. Deshalb wird im Stand der Technik das Ziel verfolgt, die Ausströmgeschwindigkeit des Fluids zu verringern und die Fluidströmung zu verwirbeln.

So beschreibt beispielsweise EP 0 612 536 eine Aortenkanüle mit niedriger Ausströmgeschwindigkeit, bei der die Verringerung der Ausströmgeschwindigkeit durch einen Diffusor in der Nähe des distalen Endes der Kanüle erreicht wird. Zum einen wird ein Diffusor in Form einer Kegelspitze beschrieben, der das Lumen am distalen Kanülenende vollständig blockiert, so dass durch die Kanüle gefördertes Blut nur durch die in der Seitenwand in der Nähe des Diffusors angeordneten Auslassöffnungen austritt. Daneben werden Diffusoren beschrieben, die mehrere Schraubenkeile aufweisen, sowie auf die Schraubenkeile abgestimmte Auslassöffnungen. Einige dieser Diffusoren blockieren die distale Öffnung des Lumens nur teilweise.

DE 196 05 864 A1 beschreibt ebenfalls eine Aortenkanüle für niedrige Ausströmgeschwindigkeiten. In diesem Fall werden die niedrigen Ausströmgeschwindigkeiten dadurch erreicht, dass in der Seitenwand der Kanüle am distalen Ende mehrere spiralförmige Schlitze ausgebildet sind, die sich in Reaktion auf Druckänderungen im Lumen der Kanüle verbreitern bzw. verengen. Die verbreiterten Schlitze führen zu einer Reduzierung der Ausströmgeschwindigkeit. Zusätzlich ist am distalen Ende der Kanüle eine zentrale Öffnung vorgesehen, die kleiner ist als der Lumendurchmesser und die zum Lumen hin kegelartig und in Ausströmrichtung trichterartig geformt ist.

EP 0 705 617 A1 beschreibt eine Aortenkanüle, bei der in der Seitenwand der blutführenden Kanüle Ausstrittsöffnungen vorgesehen sind, während das distale Ende der Kanüle durch eine Kappe blockiert ist. Zum Lumen hin ist die Kappe so ausgebildet, dass eine Ablenkung des Blutes zu den Austrittsöffnungen hin erfolgt.

DE 199 33 171 A1 beschreibt eine Kanüle zum Einbringen eines Fluids in den Aortenbogen des menschlichen Körpers, bei der der Staudruck des austretenden Freistrahls reduziert werden soll, indem in der Kanüle Mittel vorgesehen sind, die den Fluidpartikeln sowohl eine lineare Bewegung als auch eine Wirbelbewegung aufprägen. Als geeignetes Mittel wird ein Drallkörper beschrieben, hinter dem in Strömungsrichtung eine Ausgleichsstrecke oder eine abgewinkelte Strecke vorgesehen ist. Das Blut tritt dann aus dem distalen Ende der Kanüle aus einer Austrittsöffnung aus, die stirnseitig am Kanülenende liegt.

WO 99/56808 A1 beschreibt eine Aortenkanüle mit reduzierter Austrittsgeschwindigkeit, die erzielt wird, indem an einer Stelle am distalen Ende der Kanüle drei in das Innere des Lumens zurückspringende Seitenwandabschnitte vorgesehen sind. Diese Abschnitte weisen in Richtung des proximalen Endes eine Öffnung auf, so dass zum distalen Ende des Lumens strömendes Blut teilweise durch die Öffnungen abgezweigt wird und aus in der Seitenwand ausgebildeten Ausstrittsöffnungen austritt.

Vor diesem Hintergrund beschreibt die Erfindung eine Aortenkanüle zum Zuführen von Fluid, insbesondere Blut, in ein Gefäß des menschlichen Körpers, insbesondere den Aortenbogen mit einem länglich geformten Kanülengrundkörper, in dessen Inneren das Fluid geführt wird, mit einer Mündungsöffnung, aus der das Fluid am distalen Ende des Kanülengrundkörpers in einer durch den Kanülengrundkörper festgelegten Grundströmungsrichtung austritt; und mit zumindest einem ersten Ablenkelement und einem zweiten Ablenkelement, die vor der Mündungsöffnung im Strömungsweg des Fluids angeordnet sind, die jeweils eine Durchtrittsöffnung aufweisen, die in Bezug auf die Mündungsöffnung ausgerichtet ist, und die jeweils eine Ablenkfläche aufweisen, die den Teil des Fluids ablenkt, der nicht durch die Durchtrittsöffnung strömt; wobei die Durchtrittsöffnungsfläche des ersten Ablenkelements größer ist als die Durchtrittsöffnungsfläche des zweiten Ablenkelements, und / oder wobei der Ablenkwinkel der Ablenkfläche des ersten Ablenkelements größer ist als Ablenkwinkel der Ablenkfläche des zweiten Ablenkelements, das in Grundströmungsrichtung hinter dem ersten Ablenkelement angeordnet ist.

Indem sich erfindungsgemäß von einem Ablenkelement zu nächsten die Größe der Durchtrittsöffnung verkleinert und/oder der Ablenkwinkel vergrößert, wird erreicht, dass jeweils eine Teilmenge des aus der Mündungsöffnung ausströmenden Fluids aus der Grundströmung abgelenkt und seitlich abgeführt wird, wodurch die Strömungsgeschwindigkeit reduziert wird und eine Verwirbelung stattfindet. Durch die zur Mündungsöffnung ausgerichteten Durchtrittsöffnungen hindurch verläuft ein Teil des ausströmenden Fluids erfindungsgemäß weiterhin entlang der Grundströmungsrichtung, wobei aber die schrittweise Abtrennung von Teilmengen des strömenden Fluids an den Ablenkelementen auch in dem weiter in Grundströmungsrichtung strömenden Teil eine Verringerung der Geschwindigkeit hervorruft.

In einer bevorzugten Ausgestaltung der erfindungsgemäßen Aortenkanüle ist die Durchtrittsöffnungsfläche aller Ablenkelemente kleiner oder gleich der Öffnungsfläche der Mündungsöffnung. Damit sind die Ablenkelemente zumindest abschnittsweise stets in der aus der Mündungsöffnung austretenden Fluidströmung angeordnet.

In einer weiteren bevorzugten Ausgestaltung der erfindungsgemäßen Aortenkanüle ist der Gesamtdurchmesser des ersten Ablenkelements größer als die Gesamtdurchmesser des zweiten Ablenkelements, das in Grundströmungsrichtung hinter dem ersten Ablenkelement angeordnet ist. Mit anderen Worten, mit zunehmendem Abstand von der Mündungsöffnung nimmt der Gesamtdurchmesser der Ablenkelemente ab.

In einer bevorzugten Gestaltung sind die Flächeinhalte der Abelenkflächen an den verschiedenen Ablenkelementen im wesentlichen gleich. Auf diese Weise wird erreicht, dass ein ähnlich großer Anteil des Fluid von den einzelnen Ablenkelementen abgelenkt wird.

In Abstimmung mit einem in der Regel schlauchartigen Kanülengrundkörper sind das erste und zweite Ablenkelement vorteilhafterweise kreisringförmig ausgebildet.

Um eine Schädigung von durch die erfindungsgemäße Aortenkanüle gefördertem Blut zu vermeiden, sind in einer vorteilhaften Ausgestaltung der erfindungsgemäßen Aortenkanüle die der Mündungsöffnung zugewandten Oberflächen des ersten und zweiten Ablenkelements abgerundet ausgebildet.

Ein ausgewogenes Verhältnis zwischen Fertigungsaufwand und gewünschtem Effekt wird erzielt, wenn gemäß einer vorteilhaften Ausgestaltung der Aortenkanüle vier Ablenkelemente vorgesehen sind. Obwohl schon weniger als vier Ablenkelemente zu der gewünschten Beeinflussung des aus der Mündungsöffnung austretenden Fluids führen, so wird mit vier Ablenkelementen ein besseres Ergebnis erzielt. Wird die Anzahl der Ablenklemente weiter erhöht, nimmt auch der fertigungstechnischen Aufwand zu.

Das Einführen der erfindungsgemäßen Aortenkanüle in ein Gefäß wird erleichtert und die Gefahr der Beschädigung der Gefäßwand reduziert, wenn gemäß einer bevorzugten Gestaltung das am weitesten von der Mündungsöffnung angeordnete Ablenkelement auf der von der Mündungsöffnung abgewandten Seite eine abgerundete Oberfläche aufweist, die von der Durchtrittsöffnung durchdrungen wird, wodurch eine abgerundete Kanülenspitze realisiert wird.

Um die einzelnen Ablenkelemente sicher und ausgerichtet vor der Mündungsöffnung in der Fluidströmung zu positionieren, sind Fixierungselemente zur Halterung der Ablenkelemente vorgesehen, die sich von der Mündungsöffnung der Kanüle aus im wesentlichen in Grundströmungsrichtung erstrecken und an denen die Ablenkelemente fixiert sind. Die Fixierungselemente sind vorteilhafterweise schlank auszuführen, damit jede unerwünschte Beeinflussung der Fluidströmung so gering wie möglich ausfällt.

Die Fixierungselemente sind auf der dem Kanüleninneren zugewandten Seite abgerundet oder vorzugsweise tetraederförmig ausgestaltet.

Vorteilfhaft bilden die Fixierungselemente eine Außekontur zur Beabstandung der Aorteninnenwand von den Außenkanten der Ablenkelemente.

In einer bevorzugten Gestaltung sind die Ablenkelemente und die Fixierungselemente einstückig ausgebildet, was fertigungstechnische Vorteile mit sich bringt.

Dabei können entsprechend einer weiteren Ausgestaltung der erfindungsgemäßen Aortenkanüle die Ablenkelemente und die Fixierungselemente als Kanülenkopf ausgebildet werden. Dieser Kanülenkopf ist von dem Kanülengrundkörper trennbar und weist für den Kanülengrundkörper einen Befestigungsabschnitt auf. Auf diesen Befestigungsabschnitt kann der in der Regel schlauchartige Kanülengrundkörper aufgesteckt werden. Ein hohlzylindrischer Teil des Kanülenkopfes kann dann als Fortsetzung des schlauchartigen Kanülengrundkörpers angesehen werden, der die Mündungsöffnung umfasst, vor der die erfindungsgemäßen Ablenkelemente positioniert sind.

Weitere Ausgestaltungen ergeben sich aus der folgenden Beschreibung eines Ausführungsbeispiels einer erfindungsgemäßen Aortenkanüle. In der Beschreibung wird Bezug genommen auf die Zeichnungen, in denen zeigt:
- Fig. 1: eine Schnittdarstellung einer erfindungsgemäßen Aortenkanüle mit vier Ablenkelementen, die mit Fixierungselementen zu einer Kanülenspitze integriert sind;
- Fig. 2a: eine erste perspektivische Ansicht der Kanülenspitze aus Fig. 1;

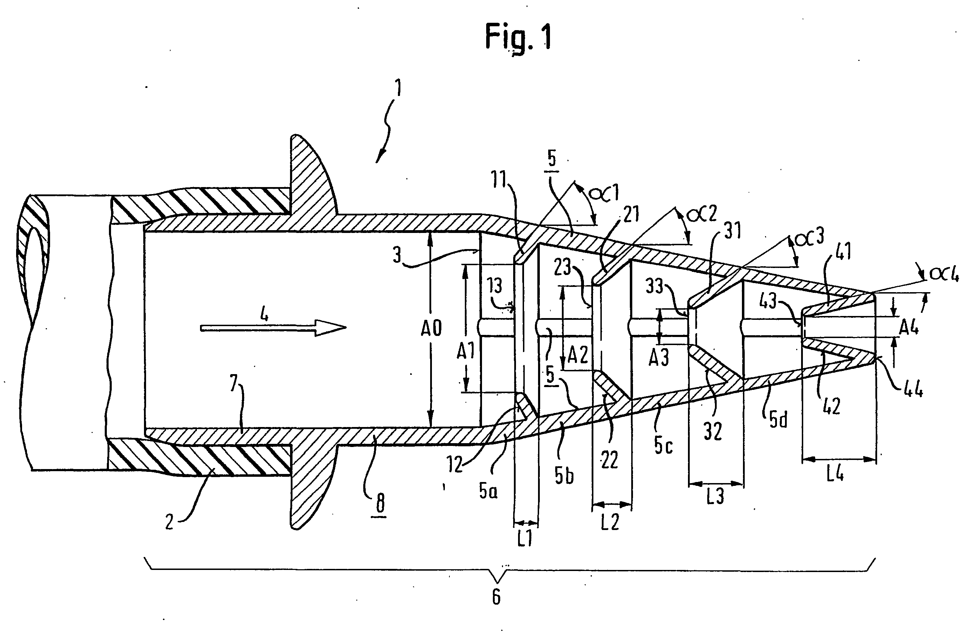

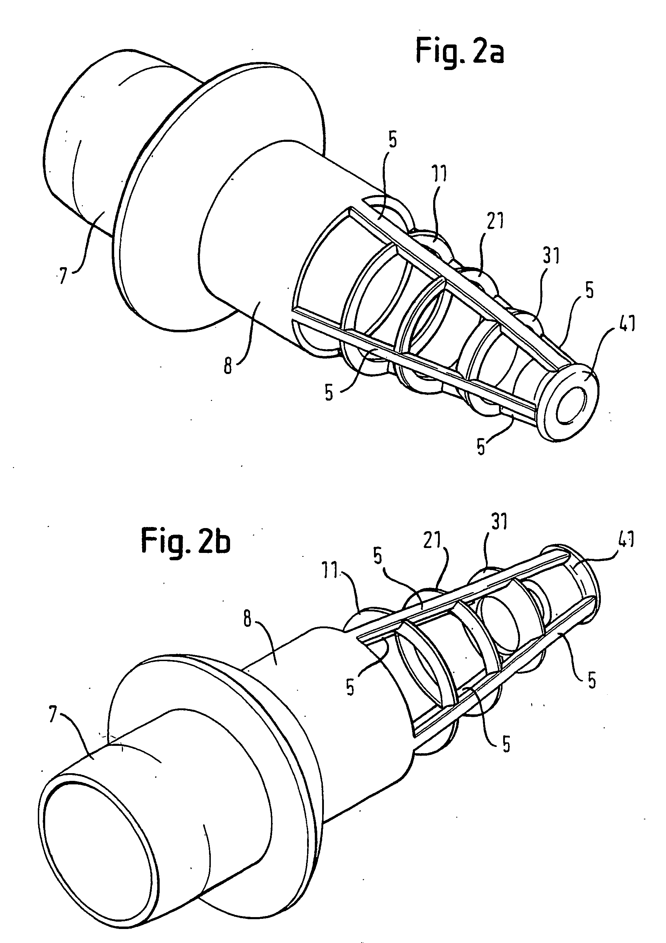

- Fig. 2a: eine zweite perspektivische Ansicht der Kanülenspitze aus Fig. 1;

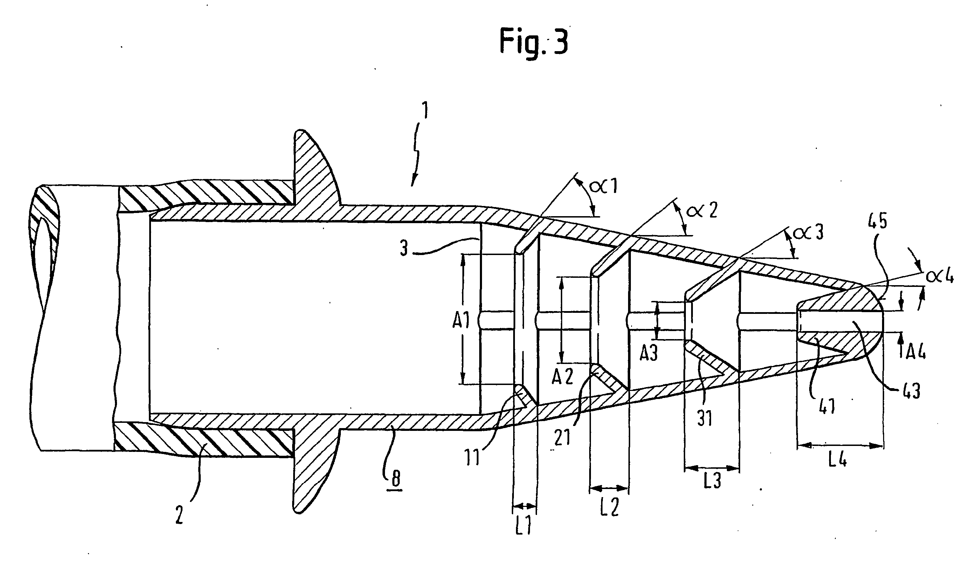

- Fig. 3: eine Teilansicht der Kanülenspitze aus Fig. 1, bei der das vorderste Ablenkelement mit einer abgerundeten Oberfläche versehen ist,

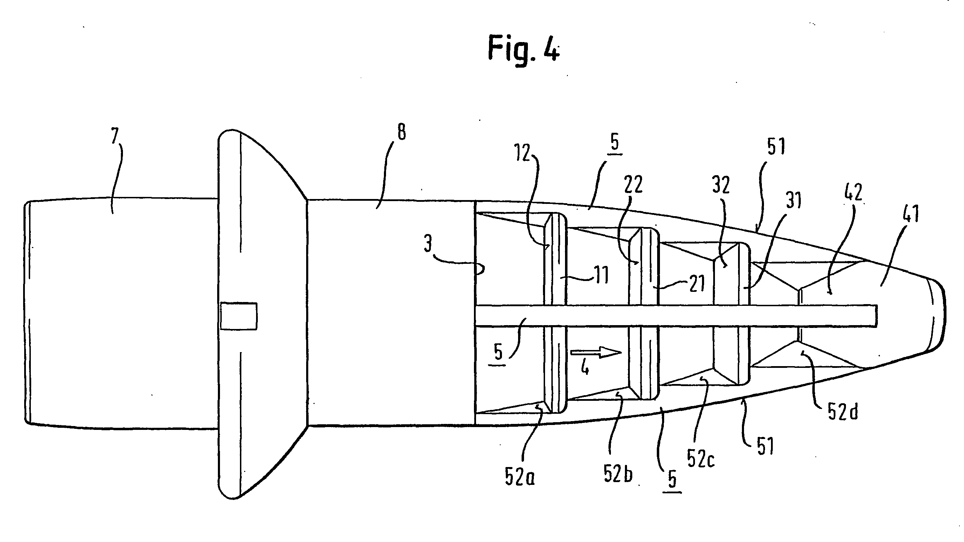

- Fig. 4: eine Seitenansicht eines zweiten Ausführungsbeispiels einer erfindungsgemäßen Aortenkanüle mit vier Ablenkelementen, die mit Fixierungselementen zu einer Kanülenspitze integriert sind;

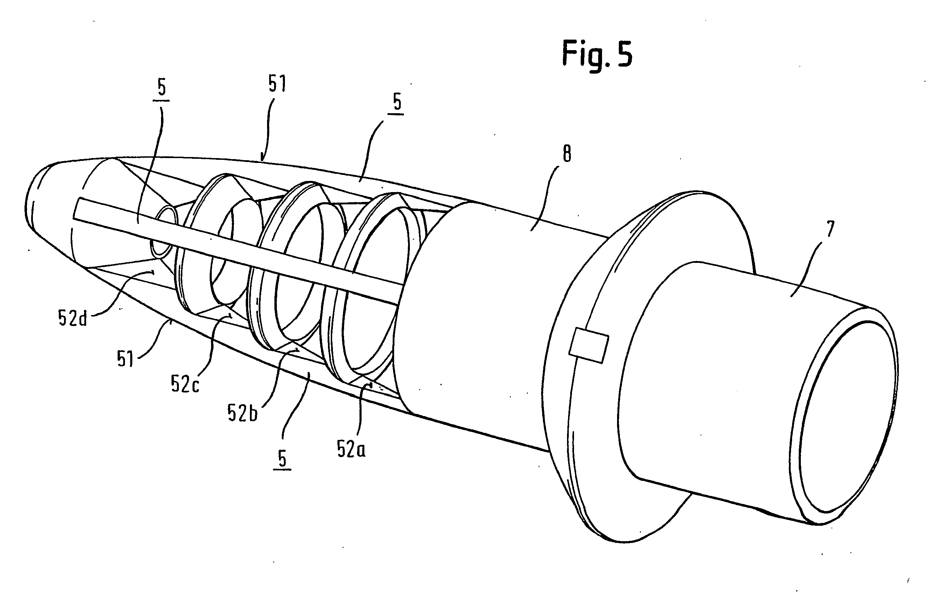

- Fig. 5: eine perspektivische Ansicht des zweiten Ausführungsbeispiels einer erfindungsgemäßen Aortenkanüle; und

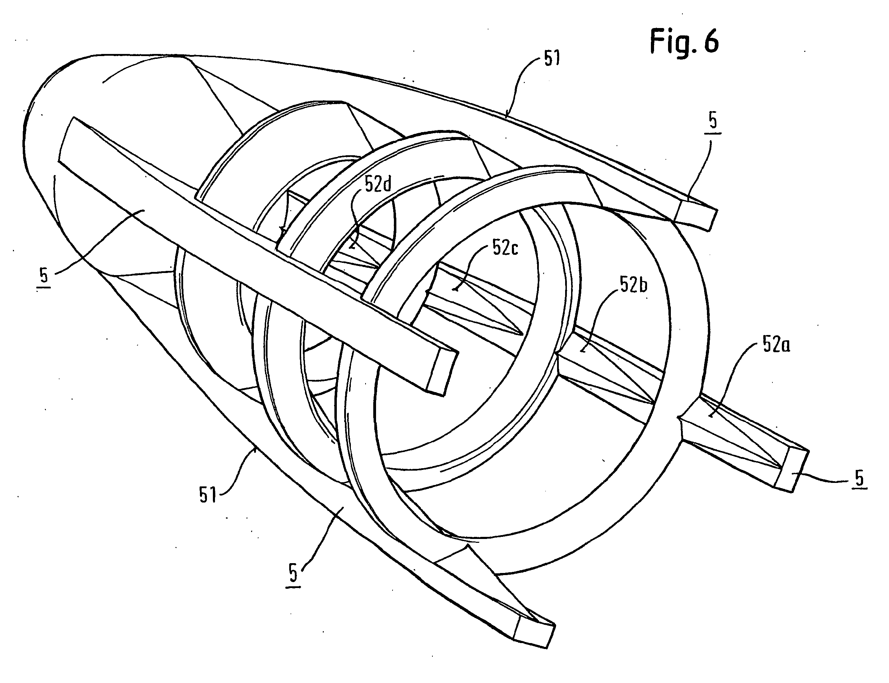

- Fig. 6: eine perspektivische Ansicht eines Teils des zweiten Ausführungsbeispiels einer erfindungsgemäßen Aortenkanüle.

In Fig. 1 ist eine erfindungsgemäße Aortenkanüle 1 zum Zuführen von Fluid, insbesondere Blut, in ein Gefäß des menschlichen Körpers, insbesondere den Aortenbogen dargestellt. Die erfindungsgemäße Aortenkanüle 1 umfasst einen länglich geformten Kanülengrundkörper 2 und 8, in dessen Inneren das Blut geführt wird. Der Kanülengrundkörper 2 und 8 weist eine Mündungsöffnung 3 auf, aus der das Blut am distalen Ende der Aortenkanüle 1in einer durch den Kanülengrundkörper festgelegten Grundströmungsrichtung 4 austritt. Ferner weist die erfindungsgemäße Aortenkanüle 1 gemäß dem Ausführungsbeispiel aus Fig. 1 ein erstes Ablenkelement 11 und ein zweites Ablenkelement 21 auf. Beide Ablenkelemente 11 und 21 sind vor der Mündungsöffnung 3 im Strömungsweg des Fluids angeordnet und besitzen jeweils eine Durchtrittsöffnung 13 bzw. 23, die in Bezug auf die Mündungsöffnung 3 ausgerichtet ist. Das zweite Ablenkelement ist 21 erfindungsgemäß in Grundströmungsrichtung 4 hinter dem ersten Ablenkelement 11 angeordnet. Ferner besitzen beide Ablenkelemente 11 und 21 jeweils eine Ablenkfläche 12 bzw. 22, die den Teil des Fluids ablenkt, der nicht durch die Durchtrittsöffnung 13 bzw. 23 strömt. Bei dem erfindungsgemäßen Ausführungsbeispiel gemäß Fig. 1 ist die Durchtrittsöffnungsfläche A1 des ersten Ablenkelements 11 größer als die Durchtrittsfläche A2 des zweiten Ablenkelements 21. Ferner ist bei dem in Fig. 1 gezeigten Ausführungsbeispiel der Erfindung der Ablenkwinkel α₁ der Ablenkfläche 12 des ersten Ablenkelements 11 größer als der Ablenkwinkel α₂ der Ablenkfläche 22 des zweiten Ablenkelements 21. Durch diese erfindungsgemäße Gestaltung der Aortenkanüle 1 wird erreicht, dass der Fluidstrahl sich sukzessive aufteilt, so dass der Volumenstrom durch den Kernbereich des vorderen Teils der Kanüle ebenso wie die Geschwindigkeit des strömenden Fluids mit jeder Stufe abnimmt.

In dieser Beschreibung eines Ausführungsbeispiels der Erfindung wird unter dem Ablenkwinkel derjenige Winkel verstanden, den die die Fluidströmung ablenkende Oberfläche eines Ablenkelements mit der Grundströmungsrichtung einnimmt. Für den Fall einer ebenen Ablenkfläche sind die Ablenkwinkel in Fig. 1 angegeben. Bei nicht-ebenen Ablenkflächen können ausgewählte Tangenten oder die Strömungsrichtung der sich einstellenden abgelenkten Fluidströmung zur Festlegung der Ablenkwinkel herangezogen werden.

Neben den bereits angesprochenen ersten und zweiten Ablenkelement 11 und 21 weist das in Fig. 1 gezeigte Ausführungsbeispiel einer erfindungsgemäßen Aortenkanüle ein drittes und ein viertes Ablenkelement 31 und 41 auf. Auch diese Ablenkelemente sind vor der Mündungsöffnung 3 im Strömungsweg des Fluids angeordnet; die Durchtrittsöffnungen 33 bzw. 43 sind im Bezug auf die Mündungsöffnung 3 ausgerichtet. In Fortsetzung der Gestaltung des ersten und des zweiten Ablenkelements 11 und 21 ist auch bei dem dritten bzw. vierten Ablenkelement 31 und 41 die Durchtrittsöffnungsfläche A3 bzw. A4 jeweils kleiner ausgelegt als die Durchtrittsöffnungsfläche des in Bezug auf die Grundströmung davor liegenden Ablenkelements. Mit anderen Worten, das in Fig. 1 gezeigte Ausführungsbeispiel einer erfindungsgemäßen Aortenkanüle 1 besitzt vier hintereinander geschaltete Ablenkelemente 11, 21, 31 und 41, bei denen die Durchtrittsöffnungsflächen A1, A2, A3 und A4 von einem Ablenkelement zum nächsten abnehmen, ebenso wie die Ablenkwinkel α₁, α₂, α₃ und α₄. Es ist offensichtlich, dass weniger als vier oder mehr als vier Ablenkelemente in identischer oder abgewandelter Anwendung dieser Gestaltungsregel eingesetzt werden können, wenn dies im Einzelfall für die Realisierung einer Aortenkanüle gemäß der Erfindung sinnvoll ist.

Aus Fig. 1 ist ferner entnehmbar, dass bei dem hier gezeigten Ausführungsbeispiel die Durchtrittsöffnungsfläche A1, A2, A3 und A4 bei allen Ablenkelementen 11, 21, 31 und 41 kleiner ist als die Öffnungsfläche A0 der Mündungsöffnung 3. Die Durchtrittsöffnungsfläche A1 des ersten Ablenkelements 11 kann aber auch gleich der Öffnungsfläche A0 der Mündungsöffnung 3 oder ein wenig größer ausgelegt sein.

Bei dem in Fig. 1 gezeigten Ausführungsbeispiel ist die Gesamtgröße der Ablenkelemente 11, 21, 31 und 41 so gewählt, dass alle Ablenkelemente, auch das erste Ablenkelement 11 kleiner ist als die Mündungsöffnung 3 des Kanülengrundkörpers. In einer vorteilhaften Ausgestaltung ist die Gesamtgröße des ersten Ablenkelements 11 gleich der Mündungsöffnung 3. Auf diese Weise ergibt sich die in Fig. 1 gezeigte Gestaltung, bei der das erste Ablenkelement 11 in seiner Gesamtgröße der Mündungsöffnung 3 im wesentlichen entspricht und die übrigen Ablenkelemente 21, 31 und 41 stufenweise kleiner werden und damit der abnehmenden Größe der Durchtrittsöffnungen A1, A2, A3 und A4 folgen.

Bei dem in Fig. 1 gezeigten Ausführungsbeispiel der Erfindung sind überdies die Ablenkelemente so gestaltet, dass deren Erstreckung L1, L2, L3, L4 in Grundströmungsrichtung 4 ebenfalls zunimmt.

Aus Fig. 1 ist ferner entnehmbar, dass in dem hier gezeigten Ausführungsbeispiel die Ablenkelemente 11, 21, 31 und 41 kreisringförmig ausgebildet sind. Dieser Umstand ergibt sich auch und insbesondere aus den perspektivischen Darstellungen 2a und 2b, auf die weiter unten noch weiter eingegangen wird.

Zur Schonung des Fluids, insbesondere des Bluts, das durch die erfindungsgemäße Aortenkanüle 1 gefördert wird und an der Mündungsöffnung 3 austritt, sind die der Mündungsöffnung 3 zugewandten Oberflächen 12, 22, 32, 42 der Ablenkelemente 11, 21, 31, 41 abgerundet ausgebildet. Diese Oberflächengestaltung ist grundsätzlich immer dann zu bevorzugen, wenn die Grundströmung des Fluids unmittelbar auf eine Oberfläche gerichtet ist. Demzufolge sind auch die der Fluidströmung zugewandten Oberflächen der im folgenden noch genauer beschriebenen Fixierungselemente 5 vorzugsweise abgerundet ausgebildet.

Bei den Fixierungselementen 5 handelt es sich bei dem hier gezeigten Ausführungsbeispiel einer erfindungsgemäßen Aortenkanüle 1 um Stege 5, an denen die Ablenkelemente 11, 21, 31 und 41 beispielhaft am äußeren Rand fixiert sind. Die Stege 5 erstrecken sich von der Mündungsöffnung 3 des Kanülengrundkörpers 2 bzw. 8 aus im wesentlichen in Grundströmungsrichtung 4. Bei dem in Fig. 1 gezeigten Ausführungsbeispiel sind vier Fixierungselemente 5 vorgesehen, von denen in der geschnittenen Darstellung der Fig. 1 lediglich drei wiedergegeben werden. In den perspektivischen Ansichten der Fig. 2a und 2b sind die vier Fixierungsstege 5 erkennbar, auch wenn sich die Stege 5 teilweise überdecken.

Fertigungstechnisch vorteilhaft ist eine einstückige Ausgestaltung der Fixierungselemente 5 und der Ablenkelemente 11, 21, 31 und 41. In Fortsetzung dieses Gedankens kann ein Kanülenkopf 6 gebildet werden, wenn die Fixierungselemente 5 überdies einstückig mit einem hohlzylindrischen Teil 8 ausgebildet werden, der einen Befestigungsabschnitt 7 für einen schlauchartigen Kanülenkörper 2 aufweist. Wenn der Kanülenschlauch 2 auf den Befestigungsabschnitt 7 aufgesteckt ist, bildet der hohlzylindrische Teil 8 des Kanülenkopfes 6 eine Verlängerung des Kanülenkörpers 2 bis zur Mündungsöffnung 3; beide Elemente zusammen sind bei dieser Ausgestaltung dann als Kanülengrundkörper anzusehen. Der Vorteil dieser Art der Gestaltung besteht darin, dass der Kanülenkopf 6, der den hohlzylindrischen Teil 8, die Fixierungselemente 5 und die Ablenkelemente 11, 21, 31 und 41 umfasst, in der Fertigung einstückig aber unabhängig von den übrigen Komponenten der Aortenkanüle hergestellt und dann auf einfache Weise sicher mit dem Kanülenschlauch 2 verbunden werden kann.

In einer weiteren Ausgestaltung der erfindungsgemäßen Aortenkanüle 1 gem. Fig. 3 ist das Ablenkelement, das in Grundströmungsrichtung am weitsten von der Mündungsöffnung entfernt angeordnet ist, im vorliegenden Fall also das vierte Ablenkelemente 41, auf der von der Mündungsöffnung 3 abgewandten Seite 44 mit einer abgerundeten Spitze 45 ausgestattet. Damit wird sichergestellt, dass beim Einführen der Aortenkanüle in ein Gefäß die Gefahr von Beschädigungen an der Gefäßinnenwand weiter verringert wird.

Obwohl sich aus den Figuren ergibt, dass bei dem hier beschriebenen Ausführungsbeispiel vier Fixierungselemente 5 vorgesehen sind, so ist ohne weiteres doch erkennbar, dass weniger oder mehr als vier Fixierungselemente 5 eingesetzt werden können. Bezüglich der sich zwischen den Ablenkelementen 11, 21, 31 und 41 ergebenden Abstandsbereichen ist anzumerken, dass die Fixierungselementabschnitte 5a, 5b, 5c, 5d in diesen Bereichen von einem Bereich zum nächsten versetzt zueinander angeordnet werden können, so dass sich entgegen den in den Figuren gezeigten Ausführungsbeispielen, keine fortlaufend geraden Fixierungselemente 5 ergeben, die von der Mündungsöffnung 3 bis zum letzten Ablenkelement 41 verlaufen. Aus den Fig. 2a und 2b ist ferner entnehmbar, dass die Fixierungsstege zudem durch die Durchtrittsöffnungen A1, A2, A3 und A4 festgelegten Innenraum hin konisch zulaufen, vorteilhafterweise jedoch ohne eine scharfe Kante zu bilden. Eine bevorzugte Ausführung ist vielmehr eine abgerundete Ausgestaltung der nach innen zulaufenden Fixierungsstege, was zu einem im wesentlichen dreieckigen Querschnitt der Stege 5 führt.

In einem konkreten Ausführungsbeispiel sind die Ablenkelemente 11, 21, 31 und 41 ca. 1,5 bis 3 mm voneinander in der Grundströmungsrichtung 4 beabstandet. Bei einer Kanüle mit ca. 8 mm Aussendurchmesser ergeben sich die Innendurchmesser und Ablenkwinkel der Ablenkelemente 11, 21, 31 und 41 beispielhaft aus der folgenden Tabelle:

| Ablenkelemente (Bezugszeichen) | Durchmesser der Durchtrittsöffnung | Ablenkwinkel |
|---|---|---|
| 11 | 5,0 - 6,5 mm | 80 - 50 |
| 21 | 4, 0 - 5, 5 mm | 60 - 40 |
| 31 | 3, 0 - 4, 5 mm | 45 - 25 |
| 41 | 1,0 - 3,5 mm | 35 - 15 |

Bei kleineren oder größeren Ausführungen müssen die Durchmesser entsprechend angepasst werden.

In Fig. 4 ist ein zweites Ausführungsbeispiel einer erfindungsgemäßen Aortenkanüle dargestellt. Wie bei dem ersten Ausführungsbeispiel umfasst die Aortenkanüle mehrere Ablenkelemente 11, 21, 31, 41, die vor der Mündungsöffnung 3 im Strömungsweg des Fluids angeordnet sind. Auch das zweite Ausführungsbeispiel besitzt Ablenkflächen 12, 22, 32, 42 auf der der Mündungsöffnung zugewandten Seite der Ablenkelemente 11, 21, 31, 41. Die Ablenkwinkel α 1, α 2, α3 und α 4 (vgl. Fig. 1), die die Ablenkflächen 12, 22, 32, 42 mit der Grundströmungsrichtung 4 einnehmen, werden kleiner mit zunehmendem Abstand des Ablenkelements 11, 21, 31, 41 von der Mündungsöffnung 3.

Bei dem zweiten Ausführungsbeispiel sind die Fixierungselemente 5 großzügiger ausgelegt. Wie sich aus Fig. 4 ergibt, reichen die Fixierungsstege 5 über die unmittelbare Ausdehnung der Ablenkelemente 11, 21, 31, 41 hinaus, wodurch eine Aussenkontour 51 gebildet wird, die sich kontinuierlich bis in die Spitze der Aortenkanüle erstrecken kann. Wie besonders deutlich in Fig. 4 ersichtlich, wird dadurch ein Körper geschaffen, der für das Einführen der Aortenkanüle vorteilhaft ist. Durch die Aussenkontur 51 der Fixierungsstege 5 wird eine Beabstandung zwischen der Aorteninnenwand und den Außenflächen bzw. -kanten der Ablenkelemente 11, 21, 31, 41 gewährleistet. Der kontinuierliche Übergang der Außenkontur 51 in die Kanülenspitze ist auch in Fig. 5 deutlich erkennbar.

Auf der dem Kanüleninneren zugewandten Seite besitzen die Fixierungsstege 5 des Ausführungsbeispiels gemäß Fig. 4 eine tetraederförmig gestaltete Oberfläche 52a, 52b, 52c, 52d. Diese Gestaltung der der Fluidströmung zugewandten Seite der Fixierungsstege 5 ist auch in Fig. 5 deutlich erkennbar. Der auf diese Flächen aufgesetzte tetraeder ist so gewählt, dass er spitz auf die Mündungsöffnung 3 zuläuft und dabei seine Höhe abnimmt. Auf der von der Mündungsöffnung 3 abgewandten Seite ist der Tetraeder jeweils bis an die Kante der Durchtrittsöffnung des jeweiligen Ablenkelements 11, 21, 31, 41 herangeführt.

Zur Verdeutlichung dieser Gestaltung ist in Fig. 6 das zweite Ausführungsbeispiel der erfindungsgemäßen Aortenkanüle ohne das hohlzylindrische Teil 8 und den Befestigungsabschnitt 7 dargestellt. Deutlich erkennbar ist die tetraederförmige Gestaltung der Oberfläche 52a, 52b, 52c, 52d auf der der Fluidströmung zugewandten Seite der Fixierungsstege 5. Auch die Außenkontur 51 der Fixierungsstege 5 ist deutlich erkennbar.

Bei beiden Ausführungsbeispielen haben die Ablenkflächen der vier Ablenkelemente denselben Flächeninhalt. Dadurch wird erreicht, dass von jedem Ablenkelement ein ähnlich großer Anteil des Fluids abgelenkt und eine gleichmäßige Verteilung der Verwirbelung über die gesamte Länge der Kanülenspitze erzielt wird.

Der hohlzylindrische Teil 8 einer erfindungsgemäßen Aortenkanüle kann auch gekrümmt ausgebildet sein, um das Einführen und Plazieren des Kanülenkopfes in das Gefäß, insbesondere in die Aorta zu erleichern.

## Patentansprüche

1. Aortenkanüle (1) zum Zuführen von Fluid; insbesondere Blut, in ein Gefäß des menschlichen Körpers, insbesondere den Aortenbogen,
a. mit einem länglich geformten Kanülengrundkörper (2,8), in dessen Inneren das Fluid geführt wird, mit einer Mündungsöffnung (3), aus der das Fluid am distalen Ende des Kanülengrundkörpers (2, 8) in einer durch den Kanülengrundkörper festgelegten Grundströmungsrichtung (4) austritt; und
b. mit zumindest einem ersten Ablenkelement (11, 21, 31) und einem zweiten Ablenkelement (21, 31, 41),
i. die vor der Mündungsöffnung (3) im Strömungsweg des Fluids angeordnet sind,
ii. die jeweils eine Durchtrittsöffnung (13, 23, 33, 43) aufweisen, die in Bezug auf die Mündungsöffnung (3) ausgerichtet ist, und
iii. die jeweils eine Ablenkfläche (12, 22, 32, 42) aufweisen, die den Teil des Fluids ablenkt, der nicht durch die Durchtrittsöffnung strömt;
iv. wobei die Durchtrittsöffnungsfläche (A1; A2; A3) des ersten Ablenkelements (11; 21; 31) größer ist als die Durchtrittsöffnungsfläche (A2; A3; A4) des zweiten Ablenkelements (21; 31; 41), und / oder
wobei der Ablenkwinkel (α₁; α₂; α₃) der Ablenkfläche (12; 22; 32) des ersten Ablenkelements (11; 21; 31) größer ist als Ablenkwinkel (α₂; α₃; α₄) der Ablenkfläche (22; 32; 42) des zweiten Ablenkelements (21; 31; 41), das in Grundströmungsrichtung (4) hinter dem ersten Ablenkelement (11; 21; 31) angeordnet ist.

2. Aortenkanüle nach Anspruch 1, **dadurch gekennzeichnet, dass** die Durchtrittsöffnungsfläche (A1, A2, A3 A4) aller Ablenkelemente (11, 21, 31, 41) kleiner oder gleich der Öffnungsfläche (A0) der Mündungsöffnung (3) ist.

3. Aortenkanüle nach Anspruch 1 oder 2, **dadurch gekennzeichnet, dass** die Gesamtgröße des ersten Ablenkelements (11; 21; 31; 41) größer ist als die Gesamtgröße des zweiten Ablenkelements (21; 31; 41), das in Grundströmungsrichtung (4) hinter dem ersten Ablenkelement angeordnet ist.

4. Aortenkanüle nach einem der vorangegangenen Ansprüche, **dadurch gekennzeichnet, dass** das erste und zweite Ablenkelement (11; 21; 31; 41) kreisringförmig ausgebildet ist.

5. Aortenkanüle nach einem der vorangegangenen Ansprüche, **dadurch gekennzeichnet, dass** die der Mündungsöffnung (3) zugewandten Oberflächen (12, 22, 32, 42) des ersten und zweiten Ablenkelements (11, 21, 31, 41) abgerundet ausgebildet sind.

6. Aortenkanüle nach einem der vorangegangenen Ansprüche, **dadurch gekennzeichnet, dass** vier Ablenkelemente (11, 21, 31, 41) vorgesehen sind.

7. Aortenkanüle nach einem der vorangegangenen Ansprüche, **dadurch gekennzeichnet, dass** die Flächeninhalte der Ablenkflächen (12, 22, 32, 42) der Ablenkelemente (11, 21, 31, 41) im wesentlichen gleich sind.

8. Aortenkanüle nach einem der vorangegangenen Ansprüche, **dadurch gekennzeichnet, dass** das am weitesten von der Mündungsöffnung angeordnete Ablenkelement (41) auf der von der Mündungsöffnung abgewandten Seite (44) eine abgerundete Oberfläche (45) aufweist, die von der Durchtrittsöffnung (43) durchdrungen wird.

9. Aortenkanüle nach einem der vorangegangenen Ansprüche, **dadurch gekennzeichnet, dass** Fixierungselemente (5) zur Halterung der Ablenkelemente (11, 21, 31, 41) vorgesehen sind, die sich von der Mündungsöffnung (3) des Kanülengrundkörpers aus im wesentlichen in Grundströmungsrichtung (4) erstrecken und an denen die Ablenkelemente (11, 21, 31, 41) fixiert sind.

10. Aortenkanüle nach Anspruch 9, **dadurch gekennzeichnet, dass** die Ablenkelemente (11, 21, 31, 41) und die Fixierungselemente (5) einstückig ausgebildet sind.

11. Aortenkanüle nach Anspruch 9, 10 oder 11, **dadurch gekennzeichnet, dass** die Fixierungselemente (5) eine Außenkontur (51) der Kanülenspitze bilden.

12. Aortenkanüle nach Anspruch 9, 10 oder 11, **dadurch gekennzeichnet, dass** die Ablenkelemente (11, 21, 31, 41) und die Fixierungselemente (5) als Kanülenkopf (6) ausgebildet sind, der einen Befestigungsabschnitt (7) für einen schlauchartigen Kanülenkörper (2) aufweist und mit dem schlauchartigen Kanülenkörper (2) trennbar verbunden ist.

13. Aortenkanüle nach Anspruch 12, **dadurch gekennzeichnet, dass** der Befestigungsabschnitt (7) einstückig mit einem hohlzylindrischen Teil (8) ausgebildet ist.

14. Aortenkanüle nach Anspruch 9, 10, 11 oder 12, **dadurch gekennzeichnet, dass** die Fixierungselemente in Form von länglichen Stegen (5) ausgebildet sind.

15. Aortenkanüle nach einem der Ansprüche 9 bis 14, **dadurch gekennzeichnet, dass** die Fixierungselemente (5) zum Kanüleninneren hin eine abgerundete Oberfläche aufweisen.

16. Aortenkanüle nach einem der Ansprüche 9 bis 14, **dadurch gekennzeichnet, dass** die Fixierungselemente (5) zum Kanüleninneren hin eine tetraeder-förmige Oberfläche aufweisen.

17. Aortenkanüle nach einem der Ansprüche 9 bis 15, **dadurch gekennzeichnet, dass** einzelne Fixierungselementeabschnitte (5a, 5b, 5c, 5d) der Fixierungselemente (5) in zwei aufeinander folgenden Abstandsbereichen zwischen Ablenkelementen (11, 21, 31, 41) versetzt zueinander angeordnet sind.

18. Kanülenkopf (6) für eine Aortenkanüle zum Zuführen von Fluid, insbesondere Blut, in ein Gefäß des menschlichen Körpers, insbesondere den Aortenbogen,
a. mit einem länglich geformten Kanülengrundkörper (7,8), in dessen Inneren das Fluid geführt wird, mit einer Mündungsöffnung (3), aus der das Fluid am distalen Ende des Kanülengrundkörpers (7, 8) in einer durch den Kanülengrundkörper festgelegten Grundströmungsrichtung (4) austritt, wobei der Kanülengrundkörper einen Befestigungsabschnitt (7) für einen schlauchartigen Kanülenkörper (2) und einen hohlzylindrischen Teil (8) umfasst; und
b. mit zumindest einem ersten Ablenkelement (11, 21, 31) und einem zweiten Ablenkelement (21, 31, 41),
i. die vor der Mündungsöffnung (3) im Strömungsweg des Fluids angeordnet sind,
ii. die jeweils eine Durchtrittsöffnung (13, 23, 33, 43) aufweisen, die in Bezug auf die Mündungsöffnung (3) ausgerichtet ist, und
iii. die jeweils eine Ablenkfläche (12, 22, 32, 42) aufweisen, die den Teil des Fluids ablenkt, der nicht durch die Durchtrittsöffnung strömt;
iv. wobei die Durchtrittsöffnungsfläche (A1; A2; A3) des ersten Ablenkelements (11; 21; 31) größer ist als die Durchtrittsöffnungsfläche (A2; A3; A4) des zweiten Ablenkelements (21; 31; 41), und / oder
wobei der Ablenkwinkel (α₁; α₂; α₃) der Ablenkfläche (12; 22; 32) des ersten Ablenkelements (11; 21; 31) größer ist als Ablenkwinkel (α 2; α 3; α 4) der Ablenkfläche (22; 32; 42) des zweiten Ablenkelements (21; 31; 41), das in Grundströmungsrichtung (4) hinter dem ersten Ablenkelement (11; 21; 31) angeordnet ist.

19. Kanülenkopf (6) für eine Aortenkanüle nach Anspruch 18, **dadurch gekennzeichnet, dass** die Durchtrittsöffnungsfläche (A1, A2, A3 A4) aller Ablenkelemente (11, 21, 31, 41) kleiner oder gleich der Öffnungsfläche (A0) der Mündungsöffnung (3) ist.

20. Kanülenkopf (6) für eine Aortenkanüle nach Anspruch 18 oder 19, **dadurch gekennzeichnet, dass** die Gesamtgröße des ersten Ablenkelements (11; 21; 31; 41) größer ist als die Gesamtgröße des zweiten Ablenkelements (21; 31; 41), das in Grundströmungsrichtung (4) hinter dem ersten Ablenkelement angeordnet ist.

21. Kanülenkopf (6) für eine Aortenkanüle nach einem der vorangegangenen Ansprüche, **dadurch gekennzeichnet, dass** das erste und zweite Ablenkelement (11; 21; 31; 41) kreisringförmig ausgebildet ist.

22. Kanülenkopf (6) für eine Aortenkanüle nach einem der vorangegangenen Ansprüche, **dadurch gekennzeichnet, dass** die der Mündungsöffnung (3) zugewandten Oberflächen (12, 22, 32, 42) des ersten und zweiten Ablenkelements (11, 21, 31, 41) abgerundet ausgebildet sind.

23. Kanülenkopf (6) für eine Aortenkanüle nach einem der vorangegangenen Ansprüche, **dadurch gekennzeichnet, dass** vier Ablenkelemente (11, 21, 31, 41) vorgesehen sind.

24. Kanülenkopf (6) für eine Aortenkanüle nach einem der vorangegangenen Ansprüche, **dadurch gekennzeichnet, dass** die Flächeninhalte der Ablenkflächen (12, 22, 32, 42) der Ablenkelemente (11, 21, 31, 41) im wesentlichen gleich sind.

25. Kanülenkopf (6) für eine Aortenkanüle nach einem der vorangegangenen Ansprüche, **dadurch gekennzeichnet, dass** das am weitesten von der Mündungsöffnung angeordnete Ablenkelement (41) auf der von der Mündungsöffnung abgewandten Seite (44) eine abgerundete Oberfläche (45) aufweist, die von der Durchtrittsöffnung (43) durchdrungen wird.

26. Kanülenkopf (6) für eine Aortenkanüle nach einem der vorangegangenen Ansprüche, **dadurch gekennzeichnet, dass** Fixierungselemente (5) zur Halterung der Ablenkelemente (11, 21, 31, 41) vorgesehen sind, die sich von der Mündungsöffnung (3) des hohlzylindrischen Teils (8) aus im wesentlichen in Grundströmungsrichtung (4) erstrecken und an denen die Ablenkelemente (11, 21, 31, 41) fixiert sind.

27. Kanülenkopf (6) für eine Aortenkanüle nach Anspruch 26, **dadurch gekennzeichnet, dass** die Ablenkelemente (11, 21, 31, 41) und die Fixierungselemente (5) einstückig ausgebildet sind.

28. Kanülenkopf (6) für eine Aortenkanüle nach Anspruch 26 oder 27, **dadurch gekennzeichnet, dass** die Fixierungselemente (5) eine Außenkontur (51) des Kanülenkopfes bilden.

29. Kanülenkopf (6) für eine Aortenkanüle nach Anspruch 26, 27 oder 28, **dadurch gekennzeichnet, dass** die Fixierungselemente in Form von länglichen Stegen (5) ausgebildet sind.

30. Kanülenkopf (6) für eine Aortenkanüle nach einem der Ansprüche 26 bis 29, **dadurch gekennzeichnet, dass** die Fixierungselemente (5) zum Kanüleninneren hin eine abgerundete Oberfläche aufweisen.

31. Kanülenkopf (6) für eine Aortenkanüle nach einem der Ansprüche 16 bis 29, **dadurch gekennzeichnet, dass** die Fixierungselemente (5) zum Kanüleninneren hin eine tetraeder-förmige Oberfläche aufweisen.

32. Kanülenkopf (6) für eine Aortenkanüle nach einem der Ansprüche 26 bis 31, **dadurch gekennzeichnet, dass** einzelne Fixierungselementeabschnitte (5a, 5b, 5c, 5d) der Fixierungselemente (5) in zwei aufeinander folgenden Abstandsbereichen zwischen Ablenkelementen (11, 21, 31, 41) versetzt zueinander angeordnet sind.

## Claims

1. Aortic cannula (1) for supplying fluid, in particular blood, to a vessel of the human body, in particular the aortic arch,
a. having an elongated shaped cannula base body (2, 8), into the interior of which the fluid is passed, having an opening (3), from which the fluid emerges at the distal end of the cannula base body (2, 8) in a basic flow direction (4) fixed by the cannula base body; and
b. having at least one first deflecting element (11, 21, 31) and a second deflecting element (21, 31, 41),
i. which are arranged upstream of the opening (3) in the flow path of the fluid,
ii. which have in each case a passage opening (13, 23, 33, 43), which is aligned with respect to the opening (3), and
iii. which have in each case a deflecting surface (12, 22, 32, 42), which deflects some of the fluid which does not flow through the passage opening;
iv. wherein the passage opening surface (A1; A2; A3) of the first deflecting element (11;21;31) is greater than the passage opening surface (A2; A3; A4) of the second deflecting element (21; 31; 41), and/or
wherein the deflecting angle (α₁; α₂; α₃) of the deflecting surface (12; 22; 32) of the first deflecting element (11; 21; 31) is greater than deflecting angle (α₂; α₃; α₄) of the deflecting surface (22; 32;
42) of the second deflecting element (21; 31; 41), which is arranged downstream of the first deflecting element (11; 21; 31) in basic flow direction (4).

2. Aortic cannula according to claim 1, **characterised in that** the passage opening surface (A1, A2, A3, A4) of all deflecting elements (11, 21, 31, 41) is smaller or the same as the opening surface (A0) of the opening (3).

3. Aortic cannula according to claim 1 or 2, **characterised in that** the overall size of the first deflecting element (11; 21; 31; 41) is greater than the overall size of the second deflecting element (21; 31; 41), which is arranged downstream of the first deflecting element in basic flow direction (4).

4. Aortic cannula according to one of the preceding claims, **characterised in that** the first and second deflecting element (11; 21; 31; 41) is designed to be annular.

5. Aortic cannula according to one of the preceding claims, **characterised in that** the surfaces (12, 22, 32, 42) facing the opening (3) of the first and second deflecting element (11, 21, 31, 41) are designed to be rounded.

6. Aortic cannula according to one of the preceding claims, **characterised in that** four deflecting elements (11,21,31,41) are provided.

7. Aortic cannula according to one of the preceding claims, **characterised in that** the areas of the deflecting surfaces (12, 22, 32, 42) of the deflecting elements (11, 21, 31, 41) are essentially the same.

8. Aortic cannula according to one of the preceding claims, **characterised in that** the deflecting element (41) arranged the furthest from the opening has a rounded surface (45) on the side (44) facing away from the opening, which rounded surface (45) is penetrated by the passage opening (43).

9. Aortic cannula according to one of the preceding claims, **characterised in that** fixing elements (5) are provided for mounting the deflecting elements (11, 21, 31, 41), which extend starting from the opening (3) of the cannula base body essentially in basic flow direction (4) and on which the deflecting elements (11,21,31,41) are fixed.

10. Aortic cannula according to claim 9, **characterised in that** the deflecting elements (11, 21, 31, 41) and the fixing elements (5) are designed to be integral.

11. Aortic cannula according to claim 9, 10 or 11, **characterised in that** the fixing elements (5) form an outer contour (51) of the cannula tip.

12. Aortic cannula according to claim 9, 10 or 11, **characterised in that** the deflecting elements (11, 21, 31, 41) and the fixing elements (5) are designed as a cannula head (6), which has an attachment section (7) for a tube-like cannula body (2) and is connected to the tube-like cannula body (2) in separable manner.

13. Aortic cannula according to claim 12, **characterised in that** the attachment section (7) is designed to be integral with a hollow cylindrical part (8).

14. Aortic cannula according to claim 9, 10, 11 or 12, **characterised in that** the fixing elements are designed in the form of elongated bars (5).

15. Aortic cannula according to one of claims 9 to 14, **characterised in that** the fixing elements (5) have a rounded surface towards the cannula interior.

16. Aortic cannula according to one of claims 9 to 14, **characterised in that** the fixing elements (5) have a tetrahedron-like surface towards the cannula interior.

17. Aortic cannula according to one of claims 9 to 15, **characterised in that** individual fixing element sections (5a, 5b, 5c, 5d) of the fixing elements (5) are arranged to be offset to one another in two sequential spacing regions between deflecting elements (11, 21, 31, 41).

18. Cannula head (6) for an aortic cannula for supplying fluid, in particular blood, to a vessel of the human body, in particular the aortic arch,
a. having an elongated shaped cannula base body (7, 8), into the interior of which the fluid is passed, having an opening (3), from which the fluid emerges at the distal end of the cannula base body (7, 8) in a basic flow direction (4) fixed by the cannula base body; wherein the cannula base body comprises an attachment section (7) for a tube-like cannula body (2) and a hollow cylindrical part (8); and
b. having at least one first deflecting element (11, 21, 31) and a second deflecting element (21, 31, 41),
i. which are arranged upstream of the opening (3) in the flow path of the fluid,
ii. which have in each case a passage opening (13, 23, 33, 43), which is aligned with respect to the opening (3), and
iii. which have in each case a deflecting surface (12, 22, 32, 42), which deflects some of the fluid which does not flow through the passage opening;
iv. wherein the passage opening surface (A1; A2; A3) of the first deflecting element (11;21;31) is greater than the passage opening surface (A2; A3; A4) of the second deflecting element (21;31; 41), and/or wherein the deflecting angle (α₁; α₂; α₃) of the deflecting surface (12; 22; 32) of the first deflecting element (11; 21; 31) is greater than deflecting angle (α₂; α₃; α₄) of the deflecting surface (22; 32; 42) of the second deflecting element (21; 31; 41), which is arranged downstream of the first deflecting element (11; 21; 31) in basic flow direction (4).

19. Cannula head (6) for an aortic cannula according to claim 18, **characterised in that** the passage opening surface (A1, A2, A3, A4) of all deflecting elements (11, 21, 31, 41) is smaller or the same as the opening surface (A0) of the opening (3).

20. Cannula head (6) for an aortic cannula according to claim 18 or 19, **characterised in that** the overall size of the first deflecting element (11; 21; 31; 41) is greater than the overall size of the second deflecting element (21; 31; 41), which is arranged downstream of the first deflecting element in basic flow direction (4).

21. Cannula head (6) for an aortic cannula according to one of the preceding claims, **characterised in that** the first and second deflecting element (11; 21; 31; 41) is designed to be annular.

22. Cannula head (6) for an aortic cannula according to one of the preceding claims, **characterised in that** the surfaces (12, 22, 32, 42) facing the opening (3) of the first and second deflecting element (11, 21, 31, 41) are designed to be rounded.

23. Cannula head (6) for an aortic cannula according to one of the preceding claims, **characterised in that** four deflecting elements (11, 21, 31, 41) are provided.

24. Cannula head (6) for an aortic cannula according to one of the preceding claims, **characterised in that** the areas of the deflecting surfaces (12, 22, 32, 42) of the deflecting elements (11, 21, 31, 41) are essentially the same.

25. Cannula head (6) for an aortic cannula according to one of the preceding claims, **characterised in that** the deflecting element (41) arranged the furthest from the opening has a rounded surface (45) on the side (44) facing away from the opening, which rounded surface (45) is penetrated by the passage opening (43).

26. Cannula head (6) for an aortic cannula according to one of the preceding claims, **characterised in that** fixing elements (5) are provided for mounting the deflecting elements (11, 21, 31, 41), which extend starting from the opening (3) of the hollow cylindrical part (8) essentially in basic flow direction (4) and on which the deflecting elements (11, 21, 31, 41) are fixed.

27. Cannula head (6) for an aortic cannula according to claim 26, **characterised in that** the deflecting elements (11, 21, 31, 41) and the fixing elements (5) are designed to be integral.

28. Cannula head (6) for an aortic cannula according to claim 26 or 27, **characterised in that** the fixing elements (5) form an outer contour (51) of the cannula head.

29. Cannula head (6) for an aortic cannula according to claim 26, 27 or 28, **characterised in that** the fixing elements are designed in the form of elongated bars (5).

30. Cannula head (6) for an aortic cannula according to one of claims 26 to 29, **characterised in that** the fixing elements (5) have a rounded surface towards the cannula interior.

31. Cannula head (6) for an aortic cannula according to one of claims 16 to 29, **characterised in that** the fixing elements (5) have a tetrahedron-like surface towards the cannula interior.

32. Cannula head (6) for an aortic cannula according to one of claims 26 to 31, **characterised in that** individual fixing element sections (5a, 5b, 5c, 5d) of the fixing elements (5) are arranged to be offset to one another in two sequential spacing regions between deflecting elements (11, 21, 31, 41).

## Revendications

1. Canule aortique (1) pour l'amenée de fluide, en particulier de sang, dans un vaisseau du corps humain, en particulier la crosse de l'aorte,
a. avec un corps de base de canule (2, 8) à forme allongée, à l'intérieur duquel le fluide est guidé, avec une ouverture d'embouchure (3), d'où le fluide sort à l'extrémité distale du corps de base de canule (2, 8), dans une direction d'écoulement de base (4) fixée par le corps de base de canule ; et
b. avec au moins un premier élément déviateur (11, 21, 31) et un deuxième élément déviateur (21, 31, 41),
i. disposés en amont de l'ouverture d'embouchure (3) dans le chemin d'écoulement du fluide,
ii. présentant chacun une ouverture de passage (13, 23, 33, 43) orientée par rapport à l'ouverture d'embouchure (3), et
iii. présentant chacun une face de déviation (12, 22, 32, 42) déviant la partie du fluide qui ne s'écoule pas à travers l'ouverture de passage,
iv. la face d'ouverture de passage (A1; A2 ; A3) du premier élément déviateur (11; 21 ; 31) étant plus grande que la face d'ouverture de passage (A2 ; A3 ; A4) du deuxième élément déviateur (21 ; 31 ; 41), et/ou
l'angle de déviation (α1 ; α2 ; α3) de la face de déviation (12 ; 22 ; 32) du premier déviateur (11 ; 21 ; 31) étant supérieur à l'angle de déviation (α2 ; α3 ; α4) de la face de déviation (22 ; 32 ; 42) du deuxième élément déviateur (21 ; 31 ; 41), qui est disposé en aval du premier élément déviateur (11 ; 21 ; 31), en observant dans la direction de l'écoulement de base (4).

2. Canule aortique selon la revendication 1, **caractérisée en ce que** la surface d'ouverture de passage (A1, A2, A3, A4) de tous les éléments déviateurs (11, 21, 31, 41) est plus petite ou identique à la face d'ouverture (A0) de l'ouverture d'embouchure (3).

3. Canule aortique selon la revendication 1 ou 2, **caractérisée en ce que** la taille globale du premier élément déviateur (11 ; 21 ; 31) est supérieure à la taille globale du deuxième élément déviateur (11 ; 21 ; 31 ; 41) est plus grande que la taille globale du deuxième élément déviateur (21 ; 31 ; 41), qui est disposé en amont du premier élément déviateur en observant dans la direction d'écoulement de base (4).

4. Canule aortique selon l'une des revendications précédentes, **caractérisée en ce que** le premier et le deuxième élément déviateur (11 ; 21 ; 31 ; 41) sont réalisés en forme d'anneau de cercle.

5. Canule aortique selon l'une des revendications précédentes, **caractérisée en ce que** les surfaces (12, 22, 32, 42), tournées vers l'ouverture d'embouchure (3), du premier et du deuxième élément déviateur (11, 21, 31, 41) sont arrondies.

6. Canule aortique selon l'une des revendications précédentes, **caractérisée en ce que** quatre éléments déviateurs (11, 21, 31, 41) sont prévus.

7. Canule aortique selon l'une des revendications précédentes, **caractérisée en ce que** les aires de surfaces des faces de déviation (12, 22, 32, 42) des éléments déviateurs (11, 21, 31, 41) sont sensiblement identiques.

8. Canule aortique selon l'une des revendications précédentes, **caractérisée en ce que** l'élément déviateur (41) disposé le plus loin de l'ouverture d'embouchure présente, sur la face (44), opposée à l'ouverture d'embouchure, une surface (45) arrondie, traversée par l'ouverture de passage (43).

9. Canule aortique selon l'une des revendications précédentes, **caractérisée en ce que** des éléments de fixation (5) sont prévus pour la fixation des éléments déviateurs (11, 21, 31, 41), qui s'étendent depuis l'ouverture d'embouchure (3) du corps de base de canule en évoluant sensiblement dans la direction d'écoulement de base (4) et sur lesquels les éléments déviateurs (11, 21, 31, 41) sont fixés.

10. Canule aortique selon revendication 9 **caractérisée en ce que** les éléments déviateurs (11, 21, 31, 41) et les éléments de fixation (5) sont réalisés d'une seule pièce.

11. Canule aortique selon la revendication 9, 10 ou 11, **caractérisée en ce que** les éléments de fixation (5) forment un contour extérieur (51) de la pointe de canule.

12. Canule aortique selon la revendication 9, 10 ou 11, **caractérisée en ce que** les éléments déviateurs (11, 21, 31, 41) et les éléments de fixation (5) sont réalisés sous la forme de têtes de canule (6), présentant un tronçon de fixation (7) pour un corps de canule (2) du type de tuyau et reliés de façon séparable au corps de canule (2) du type d'un tuyau.

13. Canule aortique selon la revendication 12, **caractérisée en ce que** le tronçon de fixation (7) est réalisé d'une seule pièce avec une partie (8) cylindrique creuse.

14. Canule aortique selon la revendication 9, 10, 11 ou 12, **caractérisée en ce que** les éléments de fixation sont réalisés sous la forme de nervures (5) allongées.

15. Canule aortique selon l'une des revendications 9 à 14, **caractérisée en ce que** les éléments de fixation (5) présentent vers l'intérieur de la canule une surface arrondie.

16. Canule aortique selon l'une des revendications 9 à 14, **caractérisée en ce que** les éléments de fixation (5) présentent vers l'intérieur de la canule une surface en forme de tétraèdre.

17. Canule aortique selon l'une des revendications 9 à 15, **caractérisée en ce que** différents tronçons d'éléments de fixation (5a, 5b, 5c, 5d) des éléments de fixation (5) sont disposés de façon décalée les uns par rapport aux autres dans deux zones d'espacement se suivant l'une l'autre entre des éléments déviateurs (11, 21, 31, 41).

18. Tête de canule (6) pour une canule aortique pour l'amenée de fluide, en particulier de sang, dans un vaisseau du corps humain, en particulier la crosse de l'aorte,
a. avec un corps de base de canule (7, 8) à forme allongée, à l'intérieur duquel le fluide est guidé, avec une ouverture d'embouchure (3), d'où le fluide sort à l'extrémité distale du corps de base de canule (7, 8), dans une direction d'écoulement de base (4) fixée par le corps de base de canule, le corps de base de canule comprenant un tronçon de fixation (7) pour un corps de canule (8) du type d'un tuyau et une partie (8) cylindrique creuse ; et
b. avec au moins un premier élément déviateur (11, 21, 31) et un deuxième élément déviateur (21, 31, 41),
i. disposés en amont de l'ouverture d'embouchure (3) dans le chemin d'écoulement du fluide,
ii. présentant chacun une ouverture de passage (13, 23, 33, 43) orientée par rapport à l'ouverture d'embouchure (3), et
iii. présentant chacun une face de déviation (12, 22, 32, 42) déviant la partie du fluide qui ne s'écoule pas à travers l'ouverture de passage,
iv. la face d'ouverture de passage (A1 ; A2 ; A3) du premier élément déviateur (11; 21; 31) étant plus grande que la face d'ouverture de passage (A2 ; A3 ; A4) du deuxième élément déviateur (21 ; 31 ; 41), et/ou
l'angle de déviation (α1 ; α2 ; α3) de la face de déviation (12 ; 22 ; 32) du premier déviateur (11 ; 21 ; 31) étant supérieur à l'angle de déviation (α2 ; α3 ; α4) de la face de déviation (22 ; 32 ; 42) du deuxième élément déviateur (21 ; 31 ; 41), qui est disposé en aval du premier élément déviateur (11 ; 21 ; 31), en observant dans la direction de l'écoulement de base (4).

19. Tête de canule (6) pour une canule aortique selon la revendication 18, **caractérisée en ce que** la surface d'ouverture de passage (A1, A2, A3, A4) de tous les éléments déviateurs (11, 21, 31, 41) est plus petite ou identique à la face d'ouverture (A0) de l'ouverture d'embouchure (3).

20. Tête de canule (6) pour une canule aortique selon la revendication 18 ou 19, **caractérisée en ce que** la taille globale du premier élément déviateur (11 ; 21 ; 31) est supérieure à la taille globale du deuxième élément déviateur (11 ; 21 ; 31 ; 41) est plus grande que la taille globale du deuxième élément déviateur (21 ; 31 ; 41), qui est disposé en amont du premier élément déviateur en observant dans la direction d'écoulement de base (4).

21. Tête de canule (6) pour une canule aortique selon l'une des revendications précédentes, **caractérisée en ce que** le premier et le deuxième élément déviateur (11 ; 21 ; 31 ; 41) sont réalisés en forme d'anneau de cercle.

22. Tête de canule (6) pour une canule aortique selon l'une des revendications précédentes, **caractérisée en ce que** les surfaces (12, 22, 32, 42), tournées vers l'ouverture d'embouchure (3), du premier et du deuxième élément déviateur (11, 21, 31, 41) sont arrondies.

23. Tête de canule (6) pour une canule aortique selon l'une des revendications précédentes, **caractérisée en ce que** quatre éléments déviateurs (11, 21, 31, 41) sont prévus.

24. Tête de canule (6) pour une canule aortique selon l'une des revendications précédentes, **caractérisée en ce que** les aires de surfaces des faces de déviation (12, 22, 32, 42) des éléments déviateurs (11, 21, 31, 41) sont sensiblement identiques.

25. Tête de canule (6) pour une canule aortique selon l'une des revendications précédentes, **caractérisée en ce que** l'élément déviateur (41) disposé le plus loin de l'ouverture d'embouchure présente, sur la face (44), opposée à l'ouverture d'embouchure, une surface (45) arrondie, traversée par l'ouverture de passage (43).

26. Tête de canule (6) pour une canule aortique selon l'une des revendications précédentes, **caractérisée en ce que** des éléments de fixation (5) sont prévus pour la fixation des éléments déviateurs (11, 21, 31, 41), qui s'étendent depuis l'ouverture d'embouchure (3) du corps de base de canule en évoluant sensiblement dans la direction d'écoulement de base (4) et sur lesquels les éléments déviateurs (11, 21, 31, 41) sont fixés.

27. Tête de canule (6) pour une canule aortique selon la revendication 26, **caractérisée en ce que** les éléments déviateurs (11, 21, 31, 41) et les éléments de fixation (5) sont réalisés d'une seule pièce.

28. Tête de canule (6) pour une canule aortique selon l'une des revendications 26 ou 27, **caractérisée en ce que** les éléments de fixation (5) forment un contour extérieur (51) de la tête de canule.

29. Tête de canule (6) pour une canule aortique selon l'une des revendications 26, 27 ou 28, **caractérisée en ce que** les éléments de fixation sont réalisés sous la forme de nervures (5) allongées.

30. Tête de canule (6) pour une canule aortique selon l'une des revendications 26 à 29, **caractérisée en ce que** les éléments de fixation (5) présentent vers l'intérieur de la canule une surface arrondie.

31. Tête de canule (6) pour une canule aortique selon l'une des revendications 16 à 29, **caractérisée en ce que** les éléments de fixation (5) présentent vers l'intérieur de la canule une surface en forme de tétraèdre.

32. Tête de canule (6) pour une canule aortique selon l'une des revendications 26 à 31, **caractérisée en ce que** différents tronçons d'éléments de fixation (5a, 5b, 5c, 5d) des éléments de fixation (5) sont disposés de façon décalée les uns par rapport aux autres dans deux zones d'espacement se suivant l'une l'autre entre des éléments déviateurs (11, 21, 31, 41).
